Europäisches Patentamt

European Patent Office

Office européen des brevets

(19)

(11) **EP 0 865 791 A1**

(12) # DEMANDE DE BREVET EUROPEEN

(43) Date de publication:
**23.09.1998 Bulletin 1998/39**

(51) Int Cl.6: **A61K 39/102**, C07K 16/12

(21) Numéro de dépôt: **98400606.4**

(22) Date de dépôt: **16.03.1998**

(84) Etats contractants désignés:
**AT BE CH DE DK ES FI FR GB GR IE IT LI LU MC NL PT SE**
Etats d'extension désignés:
**AL LT LV MK RO SI**

(30) Priorité: **17.03.1997 FR 9703198**

(71) Demandeur: **VETOQUINOL S.A.**
**70204 Lure Cedex (FR)**

(72) Inventeurs:
• **Adlam, Chris**
**70200 Magny Vernois (FR)**
• **Schreuer, Damien**
**70200 Moffans (FR)**
• **Schumacher, Catherine**
**70200 Lure (FR)**

(74) Mandataire: **Kédinger, Jean-Paul et al**
**Cabinet Malemont**
**42, avenue du Président Wilson**
**75116 Paris (FR)**

(54) **Utilisation d'une souche de Pasteurella haemolytica sérotype A6 pour la préparation d'un vaccin contre la pasteurellose bovine due à Pasteurella haemolytica**

(57)     Vaccin contre la pasteurellose bovine due à Pasteurella haemolytica, caractérisé en ce qu'il comprend une première substance antigène comprenant au moins un élément choisi dans le groupe constitué par :

(a) le surnageant, éventuellement concentré, d'une culture bactérienne de Pasteurella haemolytica sérotype A6,

(b) un extrait contenant des antigènes capsulaires de Pasteurella haemolytica sérotype A6, et

(c) des corps bactériens entiers de Pasteurella haemolytica sérotype A6, ledit surnageant, ledit extrait et lesdits corps bactériens étant biologiquement inactivés.

EP 0 865 791 A1

**Description**

La présente invention a pour objet l'utilisation d'une souche de Pasteurella haemolytica d'un sérotype particulier, pour la préparation d'un vaccin contre la pasteurellose bovine due à Pasteurella haemolytica.

Les maladies respiratoires des jeunes bovins, essentiellement du type "broncho-pneumonies infectieuses enzootiques", restent l'un des problèmes pathologiques majeurs des élevages producteurs de viandes rouge et blanche. La mortalité, les frais sanitaires et les mauvais résultats zootechniques qu'elles engendrent sont responsables de lourdes pertes économiques.

Parmi ces maladies respiratoires figure la pasteurellose bovine dont l'apparition est liée à l'intervention de deux germes bactériens, Pasteurella haemolytica et Pasteurella multocida, le premier de ces germes étant le plus fréquemment rencontré dans ces affections et le plus pathogène.

Cela étant, il convient de rappeler qu'à ce jour, on connaît deux biotypes de Pasteurella haemolytica, à savoir le biotype A et le biotype T ; la différence entre ces deux biotypes porte non seulement sur leur capacité à fermenter les sucres (biotype A : arabinose +, tréhalose - ; biotype T : arabinose -, tréhalose +), mais aussi sur leurs caractéristiques en culture, leur antibiorésistance, leur génome et leur pathogénicité.

Par ailleurs, chacun des biotypes A et T peut en outre être subdivisé en sérotypes. A ce jour, on connaît 17 sérotypes (13 de biotype A et 4 de biotype T).

Il a été procédé antérieurement par divers auteurs à des études de la distribution des sérotypes (sérotypages) de Pasteurella haemolytica à partir de prélèvements réalisés sur divers espèces d'animaux. Ainsi, par exemple :

- H.J. BALL et Coll., Br. Vet. J. (1993), 149, 561 ont montré que sur 165 prélèvements bovins de tous âges, obtenus en Irlande du Nord de 1989 à 1991, le sérotype le plus fréquemment identifié par la méthode de sérotypage par hémagglutination directe a été le sérotype 1 du biotype A ou sérotype A1 (62 prélèvements), suivi dans l'ordre de fréquence décroissante par Pasteurella haemolytica non Typable (38 prélèvements), le sérotype A2 (13 prélèvements), le sérotype T10 (12 prélèvements), le sérotype T15 (8 prélèvements), le sérotype T4 (7 prélèvements), les sérotypes A8 et A11 (6 prélèvements chacun), les sérotypes A6 et A9 (5 prélèvements chacun) et les sérotypes A14 et A13 (respectivement 2 et 1 prélèvements). On observera toutefois que dans cette étude, seuls 33 prélèvements proviennent du poumon, de sorte qu'il existe un doute sur le caractère significatif des résultats de ladite étude ; on sait en effet que comme la partie supérieure du tractus respiratoire des bovins n'est pas un environnement stérile, Pasteurella haemolytica est fréquemment présent dans la cavité nasale des animaux sains et que ce n'est que chez les animaux malades que le germe prolifère et envahit la partie profonde du tractus respiratoire ;

- M.W. ODENTAAL et M.M. HENTON, Journal of Veterinary Research, 62 : 223-226 (1995) ont étudié la distribution des sérotypes de Pasteurella haemolytica à partir de prélèvements ovins, caprins et bovins, effectués en Afrique du Sud sur une période de 8 ans, de 1986 à 1994 et montré que sur les 67 prélèvements typables sur 96, le sérotype le plus fréquemment identifié par la méthode d'hémagglutination passive a été le sérotype A1 (38 prélèvements), suivi par le sérotype A14 (8 prélèvements), le sérotype A6 (6 prélèvements) et les sérotypes A2 et A15 (5 prélèvements chacun) ;

- J.L. MARTEL et F. POUMARAT lors du Congrès de la Société Française de Buiatrie de Paris en 1988, ont présenté une étude sur les bactéries et les mycoplasmes associés aux maladies respiratoires des jeunes bovins en France et montré la prévalence du sérotype A1, cette espèce représentant environ les 2/3 des prélèvements de pasteurelles chez les bovins malades ;

- J.L. MARTEL et R. SANCHIS ont étudié la fréquence des sérotypes de Pasteurella haemolytica isolés chez les bovins, en France et présenté leurs résultats lors du Congrès annuel de la Société Française de Buiatrie du 29-30 Novembre 1995. Les souches ont été sérotypées par la méthode d'hémagglutination passive. Ils ont ainsi montré que sur les 155 souches d'origine bovine étudiées, 77 % sont de sérotype A1 et environ 6 % ne sont pas typables ; les autres souches, très minoritaires, appartiennent aux sérotypes 2 (8 %), 6 (3 %), 11 (2 %), 14 (1 %), 5 et 10(< 1 %). Les auteurs n'ont toutefois précisé ni le mode de prélèvement des souches, ni le statut pathologique de l'animal, ni même l'organe sur lequel ont été effectués les prélèvements.

Ces études antérieures montrent donc clairement que ce sont les souches Pasteurella haemolytica de sérotype A1 qui sont le plus fréquemment rencontrées chez les bovins et donc considérées comme les plus virulentes. Il n'est pas étonnant dans ces conditions que ce sont les souches de ce sérotype qui ont jusqu'ici été le plus largement utilisées dans la mise au point de vaccins contre la pasteurellose bovine.

La Demanderesse a elle-même procédé à une étude épidémiologique et a ainsi été amenée à sérotyper 107 souches de Pasteurella haemolytica d'origine bovine, de 1992 à 1995. Les résultats de cette étude sont rassemblés dans le tableau ci-après.

| Prélèvement | Sérotype A1 | Sérotype A6 | Sérotype A2 | Sérotype A9 | Non typable | Total |
|---|---|---|---|---|---|---|
| **ATT** | 23 (21,49%) | 16 (14,95%) | 2 (1,86%) | 0 (0 %) | 2 (1,86%) | 43 (40,18%) |
| **Autres £** | 15 (14,01%) | 27 (25,23%) | 2 (1,86%) | 1 (0,93%) | 9 (8,41 %) | 54 (50,46%) |
| **Inconnu $** | 6 (5,60 %) | 1 (0,93 %) | 1 (0,93%) | 1 (0,93%) | 1 (0,93%) | 10 (9,34%) |
| **Total** | 44 (41,10%) | 44 (41,10 %) | 5 (4,67) | 2 (1,86) | 12 (11,21) | 107 |
| ATT = Prélèvement par aspiration trans-traché £ = Prélèvement par écouvillonnage nasal, écouvillonnage naso-pharyngé ou sur poumon sans précision $ = Prélèvement inconnu | | | | | | |

Ces résultats montrent que si on ne fait aucune distinction au niveau du mode de prélèvement (donc du site de prélèvement), on observera la présence parmi les souches étudiées, d'autant de souches de sérotype A6 que de souches de sérotype A1.

L'écouvillonnage nasal ou naso-pharyngé reflète certes l'infection de l'animal, mais on sait que les bovins sont fréquemment des porteurs sains au niveau des voies respiratoires supérieures, et l'examen du mucus nasal reste peu représentatif de la flore pulmonaire. On observe cependant environ 25 % de prélèvements de sérotype A6 contre seulement environ 14 % de prélèvements de sérotype A1 au niveau du tractus respiratoire supérieure.

En fait, ce sont les prélèvements trachéo-bronchiques qui reflètent réellement la flore présente au niveau pulmonaire et susceptible d'induire des lésions et comme le montre la ligne ATT du tableau ci-dessus, le sérotype A1 est représenté à environ 21,5 % suivi par le sérotype A6 représenté à environ 15 %. Une proportion significative de Pasteurella haemolytica de sérotype A6 est donc trouvé au niveau du site pulmonaire, observation qui, de manière totalement inattendue, va à l'encontre des conclusions auxquelles étaient parvenus les auteurs des études passées évoquées ci-dessus. Cette différence fondamentale entre l'observation de la Demanderesse et les résultats de ces études antérieures peut s'expliquer notamment :

- par les modes de prélèvement qui, dans le plupart des cas de l'étude de la Demanderesse, ont été l'écouvillonnage naso-pharyngé ou l'aspiration trans-trachéale qui présentent la particularité de permettre des prélèvements jusque dans la partie profonde du tractus respiratoire, alors que dans les études antérieures le mode de prélèvement n'est pas précisé ou concerne essentiellement des sites correspondant à la partie supérieure du tractus respiratoire,

- par le statut pathologique des animaux sur lesquels ont été effectués les prélèvements et qui, dans le cas de l'étude de la Demanderesse, sont tous des animaux malades alors que cette information est inexistante dans certaines des études antérieures.

En vue de confirmer l'observation ci-dessus quant au rôle joué par Pasteurella haemolytica sérotype A6 dans les pasteurelloses bovines, la Demanderesse a étudié la pathogénicité de Pasteurella haemolytica sérotype A6.

On notera que jusqu'à présent, des infections expérimentales ont été effectuées avec succès sur des pré-ruminants avec Pasteurella haemolytica sérotype A1.

Il a donc été procédé à deux études dont l'objectif était d'induire une pneumonie chez de jeunes veaux par une injection unique de Pasteurella haemolytica sérotype A6 isolée du terrain, pour mettre au point un modèle d'infection expérimentale.

Plus précisément, deux veaux de trois semaines ont reçu chacun par injection trans-trachéale pour une introduction dans les bronches 25 ml d'une suspension (dans un milieu liquide maintenant les bactéries en vie, par exemple de l'eau physiologique ou un milieu de culture) contenant pour l'une environ $10^8$ UFC/ml d'une première souche de Pasteurella haemolytica sérotype A6 et pour l'autre, $10^9$ UFC/ml de cette même première souche ; par ailleurs, deux autres veaux de trois semaines ont reçu chacun par injection trans-trachéale pour une introduction dans les bronches 25 ml d'une suspension contenant pour l'une environ $10^8$ UFC/ml d'une seconde souche de Pasteurella haemolytica sérotype A6 et pour l'autre, $10^9$ UFC/ml de cette même seconde souche.

Il a été confirmé que les deux souches du terrain testées induisaient après environ 48 heures une pneumonie sévère, quelle que soit la taille de l'inoculum. Il a ainsi été démontré la nature pathogène de Pasteurella haemolytica sérotype A6.

Il s'ensuit que, contrairement à ce qui était très largement admis jusqu'à ce jour, non seulement Pasteurella haemolytica sérotype A1, mais également Pasteurella haemolytica sérotype A6 sont tous deux des agents pathogènes majeurs des voies respiratoires bovines, avec bien entendu la possibilité d'utiliser Pasteurella haemolytica sérotype A6 ou Pasteurella haemolytica sérotype A6 conjointement avec Pasteurella haemolytica sérotype A1 pour la mise au point d'un vaccin.

L'un des objets de la présente invention est donc l'utilisation de certains éléments spécifiques d'une souche de Pasteurella haemolytica sérotype A6 ou de certains éléments spécifiques d'une souche de Pasteurella haemolytica sérotype A6 conjointement avec une souche de Pasteurella haemolytica sérotype A1, pour la préparation d'un vaccin contre la pasteurellose bovine due à Pasteurella haemolytica.

On pourra ainsi disposer d'un vaccin efficace non seulement pour prévenir les pasteurelloses liées à l'intervention de Pasteurella haemolytica sérotype A1, mais également celles liées à l'intervention de Pasteurella haemolytica sérotype A6 ; un tel vaccin permettra donc de protéger l'espèce bovine de manière beaucoup plus étendue qu'avec les vaccins existant à ce jour et dont la conception est basée sur la seule souche Pasteurella haemolytica sérotype A1.

Ainsi, un objet de la présente invention est un vaccin contre la pasteurellose bovine due à Pasteurella haemolytica, ce vaccin étant caractérisé en ce qu'il comprend une première substance antigène comprenant au moins un élément choisi dans le groupe constitué par :

(a) le surnageant, éventuellement concentré, d'une culture bactérienne de Pasteurella haemolytica sérotype A6,
(b) un extrait contenant des antigènes capsulaires de Pasteurella haemolytica sérotype A6, et
(c) des corps bactériens entiers de Pasteurella haemolytica sérotype A6, ledit surnageant, ledit extrait et lesdits corps bactériens étant biologiquement inactivés.

Par ailleurs, le vaccin selon l'invention peut en outre comprendre une seconde substance antigène comprenant au moins un élément choisi dans le groupe constitué par :

(a') le surnageant, éventuellement concentré, d'une culture bactérienne de Pasteurella haemolytica sérotype A1,
(b') un extrait contenant des antigènes capsulaires et/ou de membrane de Pasteurella haemolytica sérotype A1,
(c') des corps bactériens entiers de Pasteurella haemolytica sérotype A1, et
(d') des fractions de tels corps bactériens,

ledit surnageant, ledit extrait, lesdits corps bactériens et lesdites fractions étant biologiquement inactivés.

Dans la mesure où les corps bactériens entiers susmentionnés sont susceptibles de présenter une certaine toxicité intrinsèque, il est préférable selon l'invention qu'ils soient présents en faible quantité dans le vaccin.

Selon une variante de l'invention, ladite première substance antigène est constituée par le surnageant biologiquement inactivé et concentré d'une culture de Pasteurella haemolytica sérotype A6.

Selon une autre variante de l'invention, ladite seconde substance antigène est constituée par le surnageant biologiquement inactivé et concentré d'une culture de Pasteurella haemolytica sérotype A1.

Selon une variante particulièrement préférée de l'invention, le vaccin comprend une première substance antigène constituée par le surnageant biologiquement inactivé et concentré d'une culture de Pasteurella haemolytica sérotype A6, et une seconde substance antigène constituée par le surnageant biologiquement inactivé et concentré d'une culture de Pasteurella haemolytica sérotype A1.

Selon l'efficacité souhaitée pour le vaccin, ce dernier peut en outre comprendre un ajout de leucotoxine de Pasteurella haemolytica, autre que la leucotoxine éventuellement déjà présente dans ladite première substance antigène et/ou dans ladite seconde substance antigène. Cette leucotoxine pourra par exemple être de la leucotoxine recombinante ou de la leucotoxine obtenue par purification à partir d'un surnageant de culture bactérienne de Pasteurella haemolytica sérotype A6 et/ou de Pasteurella haemolytica sérotype A1.

Selon une autre variante encore, l'extrait contenant des antigènes capsulaires de Pasteurella haemolytica sérotype A6 et l'extrait contenant des antigènes capsulaires de Pasteurella haemolytica sérotype A1 sont respectivement ceux obtenus par extraction de cellules de Pasteurella haemolytica sérotype A6 ou A1 par une solution aqueuse d'un sel minéral ou organique.

Il est à noter que les antigènes capsulaires susmentionnés sont du type polysaccharide et que le sel utilisé pour l'extraction est par exemple du chlorure de sodium ou du salicylate de sodium.

On précisera que l'inactivation dudit (desdits) surnageant(s), extrait(s) et corps bactériens entiers et desdites fractions des corps bactériens susmentionnés peut être effectuée par n'importe quel moyen bien connu dans la technique antérieure ; il pourra ainsi s'agir par exemple d'une inactivation chimique, de préférence par le formaldéhyde ou le phénol, ou d'une inactivation thermique.

Il est avantageux, mais non obligatoire, pour renforcer l'efficacité du vaccin, d'incorporer au moins un adjuvant d'immunisation dans le vaccin selon l'invention. Cet adjuvant peut être n'importe quel adjuvant d'immunisation habituellement utilisé dans la technique antérieure et on citera à titre d'exemples, l'hydroxyde d'aluminium sous forme de gel (par exemple celui disponible sous la marque ALHYDROGEL® auprès de la société danoise SUPERFOS A/S) et une saponine telle que la saponine de quillaja (par exemple celle disponible sous la marque QUIL-A auprès de la société danoise SUPERFOS A/S). Cet adjuvant peut être utilisé à diverses concentrations que l'homme de métier pourra aisément établir.

On notera que dans le cas où le vaccin comprend un concentré du surnageant d'une culture de Pasteurella haemolytica sérotype A6, un concentré du surnageant d'une culture de la souche Pasteurella haemolytica sérotype A1 et éventuellement un ajout de leucotoxine de Pasteurella haemolytica, celui-ci a fait preuve d'une bonne efficacité lorsque la quantité du premier concentré est telle que son activité leucotoxique 50 % soit d'au moins 8 unités et la quantité du second concentré est telle que son activité leucotoxique 50 % soit d'au moins 8 unités.

La définition de l'activité leucotoxique 50 % sera explicitée ci-après, étant précisé que les activités seuils ci-dessus de 8 unités ne sont données qu'à titre illustratif de l'invention et que des activités inférieures à ces seuils entrent parfaitement dans la portée de la présente invention.

La présente invention s'étend par ailleurs à tout concentré du surnageant d'une culture bactérienne de Pasteurella haemolytica sérotype A6, ce concentré ou surnageant étant ou non biologiquement inactivé.

La présente invention s'étend encore à un procédé de préparation d'un vaccin dans lequel la première et la seconde substances antigènes sont des surnageants concentrés de culture bactérienne. Ce procédé comprend de préférence les opérations de :

(a) culture d'une souche-mère de Pasteurella haemolytica sérotype A6,
(b) culture séparée d'une souche-mère de Pasteurella haemolytica sérotype A1,
(c) séparation par filtration d'une partie au moins des cellules bactériennes du surnageant obtenu par l'opération (a) ci-dessus,
(d) séparation par filtration d'une partie au moins des cellules bactériennes du surnageant obtenu par l'opération (b) ci-dessus,
(e) concentration du surnageant obtenu par l'opération (c) et du surnageant obtenu par l'opération (d) ci-dessus,
(f) mélange des concentrés obtenus par l'opération (e) ci-dessus dans une proportion appropriée,
(g) inactivation biologique des deux concentrés obtenus par l'opération (e) ci-dessus, avant ou après leur mélange par l'opération (f) ci-dessus,
(h) adjonction éventuelle d'un ou plusieurs adjuvants d'immunisation au mélange de concentrés inactivés obtenu précédemment,
(i) adjonction éventuelle de leucotoxine de Pasteurella haemolytica, et
(j) réglage éventuel du pH du mélange obtenu par l'opération (i) ci-dessus à la valeur souhaitée.

La présente invention s'étend enfin à un procédé de préparation d'un vaccin dans lequel la première et la seconde substances antigènes sont des extraits contenant des antigènes capsulaires. Un tel procédé comprend de préférence les opérations de :

(a) culture d'une souche-mère de Pasteurella haemolytica sérotype A6 et culture séparée d'une souche-mère de Pasteurella haemolytica sérotype A1,
(b) collecte des cellules bactériennes obtenues par l'opération (a) ci-dessus,
(c) extraction par une solution aqueuse d'un sel minéral ou organique respectivement des cellules collectées à l'opération (b),
(d) élimination des matières cellulaires pour récupérer un extrait aqueux contenant des antigènes capsulaires de Pasteurella haemolytica sérotype A6 et un extrait aqueux contenant des antigènes capsulaires de Pasteurella haemolytica sérotype A1,
(e) purification de chacun des extraits,
(f) mélange des extraits purifiés,
(g) inactivation biologique desdits extraits avant ou après leur mélange par l'opération (f) ci-dessus,
(h) adjonction éventuelle d'un ou plusieurs adjuvants d'immunisation au mélange des extraits inactivés obtenu précédemment,
(i) adjonction éventuelle de leucotoxine de Pasteurella haemolytica, et
(j) réglage éventuel du pH du mélange obtenu par l'opération (i) ci-dessus à la valeur souhaitée.

On précisera que dans ce dernier procédé, il est avantageux que :

- la culture des souches-mères soit effectuée comme décrit ci-après,
- la collecte des cellules bactériennes soit effectuée par centrifugation ou filtration,
- l'extraction soit mise en oeuvre à l'aide d'une solution aqueuse de chlorure de sodium ou de salicylate de sodium (de préférence à une concentration d'environ 2,5 %) avec agitation (à 22° C ou plus),
- l'élimination des matières cellulaires soit effectuée par centrifugation à grande vitesse et la récupération des extraits aqueux soit effectuée par dialyse contre de l'eau distillée, et
- la purification desdits extraits soit effectuée par traitement enzymatique (ribonucléase, désoxyribonucléase et/ou protéinase K) pour éliminer les traces d'acides nucléiques et les protéines indésirables, suivi de la précipitation des antigènes capsulaires (polysaccharides) par utilisation par exemple de trois volumes d'éthanol à 95 % (ou tout autre solvant approprié comme l'acétone par exemple), redissolution du précipité formé dans de l'eau, puis nouvelle précipitation par le bromure de cétyltriméthylammonium, après quoi le complexe précipité est dissous dans NaCl 2M, et soumis à une dialyse poussée contre de l'eau, une ultracentrifugation (par exemple à 105 000 x g) pour éliminer les liposaccharides indésirables et une purification finale par gel filtration ou échange d'ions.

On notera encore qu'une récupération supplémentaire d'antigènes capsulaires peut être obtenue de la manière suivante : on soumet les matières cellulaires éliminées lors de l'opération (d) mentionnée ci-dessus, à une extraction par un mélange chaud phénol-eau ; la phase aqueuse obtenue est ensuite dialysée contre de l'eau jusqu'à ce qu'elle soit exempte de phénol, puis lyophilisée ; le produit de lyophilisation est purifié comme ci-dessus, à savoir ultracentrifugation et gel filtration ou échange d'ions.

L'invention sera maintenant illustrée par les éléments techniques suivants donnés à titre nullement limitatif de l'invention, ces éléments se rapportant à la production et à l'étude d'efficacité d'un vaccin comprenant, à titre de première et seconde substances antigènes, des surnageants de culture bactérienne de Pasteurella haemolytica sérotype A6 et de Pasteurella haemolytica sérotype A1.

## I/ Production d'un vaccin

### 1/ Souches utilisées

Utilisation a été faite d'une souche de Pasteurella haemolytica sérotype A1 et d'une souche de Pasteurella haemolytica sérotype A6, toutes deux d'origine bovine, et dont le sérotype peut notamment être réalisé par la méthode bien connue d'hémagglutination passive.

Il est à noter toutefois qu'il peut être fait utilisation de n'importe quelle souche Pasteurella haemolytica sérotype A6 et de n'importe quelle souche Pasteurella haemolytica sérotype A1, la seule condition qu'elles doivent remplir étant qu'elles soient de sérotype A6 et de sérotype A1 respectivement. Ainsi, utilisation peut être faite de n'importe quelle souche provenant du terrain après avoir vérifié par une opération classique de sérotypage qu'elle appartient au sérotype recherché, ou provenant d'une collection [comme par exemple l'American Type Culture Collection (ATCC)].

### 2/ Multiplication des souches

Chaque souche est utilisée pour ensemencer une gélose (DSA = Dextrose Starch Agar) en boîtes de Pétri, avec incubation à 37° C, sous $CO_2$ (5 %) jusqu'à apparition de colonies visibles à l'oeil nu (généralement 12 à 14 heures). Chaque gélose résultante est utilisée pour ensemencer un milieu gélosé (DSA) coulé en bouteilles de culture cellulaire, ce milieu gélosé étant ensuite incubé 15 à 18 heures à 37° C, dans une atmosphère à 5 % de $CO_2$

### 3/ Culture de production

La suspension récoltée, pour chaque souche, à partir des bouteilles de culture cellulaire est ensuite utilisée pour ensemencer un fermentateur utilisant, à titre de milieu de culture, du milieu RPMI 1640 contenant 5 % de DSB (Dextrose Starch Broth) ou un bouillon d'infusion de coeur-cerveau ou un bouillon de DSB ou encore un milieu de culture solide bien connu dans la technique considérée. On procède à la culture à 37° C jusqu'en fin de la phase logarithmique de croissance. On précisera que d'autres milieux de culture pourront être utilisés parmi ceux actuellement disponibles sur le marché.

Ces opérations peuvent être répétées jusqu'à obtention de la quantité souhaitée de surnageant.

### 4/ Récolte

Pour chaque souche, les cellules bactériennes sont ensuite séparées du surnageant par exemple par filtration sur des membranes présentant une ouverture de mailles de 0, 22 μm.

5/ Dosage de la leucotoxine

L'efficacité du vaccin étant fonction notamment de son titre en leucotoxines qui sont des antigènes produits lors de la culture des souches Pasteurella haemolytica mises en oeuvre, il convient de soumettre les surnageants susmentionnés à des concentrations suffisantes pour atteindre les activités leucotoxiques souhaitées. Cette activité est donc dosée sur les surnageants afin de déterminer à quelles concentrations il faut les soumettre pour obtenir les activités leucotoxiques finales voulues.

Le principe de ce dosage est le suivant :

L'activité leucotoxique est déterminée par un test sur microplaque utilisant des cellules BL3 (pour Bovine Leukemia Cell provenant de la collection de l'ATCC où elles sont enregistrées sous le code 8037-CRL). Des cellules équivalentes sensibles à la leucotoxine peuvent également être utilisées.

Ces cellules sont incubées (1 heure, 37° C) en présence de différentes dilutions de l'échantillon (surnageant) à titrer (pur, 1/2, 1/4, 1/8, 1/16 ... 1/64). Les cellules survivantes à la fin de la période d'incubation sont révélées par coloration au rouge neutre. Après solubilisation des cellules, la coloration est titrée au spectrophomètre à 550nm. Le pourcentage d'activité toxique est déterminé pour chaque dilution de la manière suivante :

$$\% \text{ de toxicité} = \frac{A - B}{A} \times 100$$

dans laquelle :

A = densité optique moyenne de 4 puits de contrôle contenant seulement du milieu de culture (cette préparation n'étant pas toxique, la totalité des cellules BL3 survivent), et
B = densité optique moyenne de 4 puits test (échantillon à tester).

Le % de toxicité est calculé pour chaque dilution de l'échantillon à titrer.

On s'aperçoit qu'au fur et à mesure qu'on dilue l'échantillon à titrer, sa toxicité diminue (car on dilue la leucotoxine responsable de la toxicité).

En représentant graphiquement la toxicité en fonction de la dilution, on obtient une courbe à partir de laquelle on peut déduire la dilution la plus forte de l'échantillon, donnant encore au moins 50 % de toxicité.

Ainsi, si par exemple la dernière dilution donnant encore au moins 50 % de toxicité est la dilution 1/2, l'activité de l'échantillon testé (surnageant avant concentration) est de 2 unités (en fait l'inverse de la dilution). Ceci signifie que si une valeur de 8 unités est souhaitée pour l'activité leucotoxique du vaccin, il faudra concentrer le surnageant entre 4 et 8 fois pour espérer atteindre le titre de 8 unités souhaité.

6/ Concentration du surnageant

Le surnageant mentionné aux points 4/ et 5/ ci-dessus, est concentré au degré voulu par passage sur des membranes d'une porosité correspondant à des poids moléculaires de 1 à 10 kD.

7/ Inactivation biologique

Chaque concentré ainsi obtenu (concentré correspondant à la souche de sérotype A1 et concentré correspondant à la souche de sérotype A6) est ensuite inactivé par addition d'une solution aqueuse à 40 % de formaldéhyde et incubation à 37° C, sous agitation et pendant au moins 24 heures. La quantité de solution aqueuse de formaldéhyde sera généralement de 0,1 à 0,5 % (V/V) par rapport au concentré. Ensuite, le formaldéhyde est neutralisé par addition d'une solution de métabisulfite de sodium.

8/ Mélange

Les deux concentrés inactivés ainsi obtenus sont ensuite mélangés dans la proportion souhaitée. On fait suivre par l'addition d'ALHYDROGEL (par exemple à la dose de 7,5 mg/ml, ce qui correspond à 25 volumes d'un gel aqueux à 3 % d'hydroxyde d'aluminium pour 75 volumes de vaccin sans gel) et de QUIL-A (par exemple à la dose de 0,05 mg/ml, ce qui correspond à 0,0033 ml d'une solution mère de QUIL-A à 15 mg/ml d'eau, par ml de vaccin final), l'ajustement du pH à 6,5-8,0 à l'aide d'une solution aqueuse à 7,5 % de bicarbonate de sodium, de soude ou d'acide chlorhydrique et le conditionnement en ampoules stériles.

9/ Exemple de composition du vaccin

Un exemple de composition du vaccin selon l'invention est comme suit :

- 36 % en volume de concentré, inactivé et neutralisé, provenant de la culture de Pasteurella haemolytica sérotype A1, ayant avant inactivation une activité leucotoxique 50 % d'au moins 8 unités,
- 36 % en volume de concentré, inactivé et neutralisé, provenant de la culture de Pasteurella haemolytica sérotype A6, ayant avant inactivation une activité leucotoxique 50 % d'au moins 8 unités,
- 25 % en volume de gel aqueux à 3 % d'hydroxyde d'aluminium,
- 0,33 % en volume de solution mère de QUIL-A, et
- 2,67 % en volume de solution à 7,5 % de bicarbonate de sodium.

**II/ Etude de l'efficacité du vaccin**

A/ Démonstration qu'un vaccin contenant les surnageants de culture respectivement d'une souche de Pasteurella haemolytica sérotype A6 et d'une souche de Pasteurella haemolytica sérotype A1 est capable de protéger des veaux pré-ruminants contre une épreuve par des organismes virulents Pasteurella haemolytica sérotype A6.

Pour cette démonstration, des groupes de jeunes veaux ont été vaccinés par voie intramusculaire avec deux doses de préparation vaccinales ou de placebo comme suit :

Groupe 1 :  placebo
Groupe 2 :  vaccin du commerce
Groupe 3 :  vaccin selon l'invention (mélange dilué à 75 % d'un concentré du surnageant de culture bactérienne de souche Pasteurella haemolytica sérotype A6 et d'un concentré de surnageant de culture bactérienne de souche Pasteurella haemolytica sérotype A1),
Groupe 4 :  vaccin selon l'invention, à savoir mélange d'un concentré du surnageant de culture bactérienne de souche Pasteurella haemolytica sérotype A6 et d'un concentré de surnageant de culture bactérienne de souche Pasteurella haemolytica sérotype A1.

Les vaccinations ont été effectuées avec des intervalles de 21 jours entre les injections et tous les animaux ont été éprouvés par voie intra-trachéale avec une culture de Pasteurella haemolytica sérotype A6, 7 jours après la seconde injection de vaccin (ou de placébo).

La dose d'épreuve a été de 25 ml de la culture susvisée au stade phase logarithmique et contenant $1,7 \times 10^8$ cellules par ml. Avant et après l'épreuve, les animaux ont été évalués sur le plan clinique selon un système d'évaluation et après décès éventuel, les poumons ont été examinés en vue d'estimer le degré d'infection.

Les résultats obtenus sont rassemblés dans le Tableau 1 ci-après.

Tableau 1

| Groupe | Décès | Evaluation clinique (*) | Evaluation des poumons | | Lobes infectés | Poids des poumons (kg) |
|--------|-------|-------------------------|------------------------|--|----------------|-------------------------|
| | | | Gravité/Surface | | | |
| 1 | 3/10 | 40 | 21,5 | 13,8 | 5,8 | 1,36 |
| 2 | 2/8 | 29 | 12,1 | 12,3 | 5,0 | 1,32 |
| 3 | 1/9 | 21 | 13,2 | 10,1 | 4,3 | 1,17 |
| 4 | 1/10 | 19 | 11,2 | 8,2 | 4,6 | 0,94 |

(*) Les résultats sont des moyennes pour le groupe entier.

Ce tableau montre qu'en terme de mortalité, d'évaluation clinique, d'évaluation des poumons et de poids des poumons, les vaccins selon l'invention (Groupes 3 et 4) sont supérieurs au placebo (Groupe 1). Le vaccin du commerce (Groupe 2) s'est également mieux comporté que le placebo, mais n'était pas aussi efficace que les vaccins selon l'invention; on notera que ce vaccin du commerce ne contenait pas de composant sérotype A6.

Démonstration qu'un vaccin contenant les surnageants de culture respectivement d'une souche Pasteurella haemolytica sérotype A6 et d'une souche Pasteurella haemolytica sérotype A1 est capable de protéger des veaux ruminants contre une épreuve par un organisme virulent Pasteurella haemolytica sérotype A6.

Une étude à une échelle plus réduite a été effectuée pour tester les capacités protectrices du vaccin selon l'invention chez des groupes de veaux plus âgés. Il est souvent difficile de reproduire la pasteurellose chez de tels animaux, car il existe en fait une exposition naturelle aux organismes Pasteurella haemolytica, laquelle entraîne un certain degré de résistance à l'épreuve. Cependant, dans cette expérience, une pathologie a été produite, ce qui une nouvelle fois démontre la virulence de la souche sérotype A6 utilisée pour l'épreuve.

Deux groupes de quatre veaux ruminants ont reçu, par voie intramusculaire, soit deux doses de vaccin selon l'invention (Groupe 1), soit deux doses de placebo (Groupe 2).

Des doses de vaccin ou de placebo ont été administrées à trois semaines d'écart et, deux semaines après l'administration de la seconde dose de vaccin ou de placebo, les animaux ont été éprouvés par voie intra-trachéale avec 40 ml d'une culture au stade phase logarithmique d'une souche Pasteurella haemolytica sérotype A6 et contenant $1,35 \times 10^9$ organisme par ml.

Les animaux ont été sacrifiés une semaine après l'épreuve. Ils ont été suivis cliniquement avant et après l'épreuve et les lésions de leurs poumons ont été caractérisées et évaluées par autopsie.

Deux veaux additionnels (Groupe 3) ont servi de témoins non vaccinés et non éprouvés. Les résultats obtenus sont rassemblés dans le tableau 2 ci-après :

Tableau 2

| Groupe | Evaluation des poumons | | Lobes infectés | Poids des poumons (g) | Evaluation clinique totale |
|---|---|---|---|---|---|
| | Gravité-surface | | | | |
| 1 | 9,8±4,5 | 7,0±2,9 | 3,0±1,4 | 2521±66,8 | 4 |
| 2 | 22,8±10,0 | 16,0±6,5 | 6,0±2,2 | 3389±1249,0 | 6 |
| 3 | 5,0±7,0 | 2,5±3,5 | 2,5±3,5 | 2083±97,6 | 0 |

Il est à noter que dans cette expérimentation, tous les animaux ont survécu à l'épreuve.

Comme cela est montré par le degré de dommages des poumons et les réponses cliniques, les animaux du Groupe 1 ayant reçu le vaccin selon l'invention sont protégés par comparaison avec ceux du Groupe 2 ayant reçu le placebo.

C/ Conclusion

L'étude ci-dessus montre que le sérotype A6 de Pasteurella haemolytica est virulent à la fois pour les veaux pré-ruminants et les veaux ruminants.

Par ailleurs, un vaccin contenant le surnageant, éventuellement concentré, de culture de Pasteurella haemolytica sérotypes A6 et A1 a protégé les veaux pré-ruminants et ruminants quant ils ont été éprouvés par Pasteurella haemolytica sérotype A6.

**Revendications**

1. Vaccin contre la pasteurellose bovine due à Pasteurella haemolytica, caractérisé en ce qu'il comprend une première substance antigène comprenant au moins un élément choisi dans le groupe constitué par :

    (a) le surnageant, éventuellement concentré, d'une culture bactérienne de Pasteurella haemolytica sérotype A6,
    (b) un extrait contenant des antigènes capsulaires de Pasteurella haemolytica sérotype A6, et
    (c) des corps bactériens entiers de Pasteurella haemolytica sérotype A6, ledit surnageant, ledit extrait et lesdits corps bactériens étant biologiquement inactivés.

2. Vaccin selon la revendication 1, caractérisé en ce qu'il comprend en outre une seconde substance antigène comprenant au moins un élément choisi dans le groupe constitué par :

    (a') le surnageant, éventuellement concentré, d'une culture bactérienne de Pasteurella haemolytica sérotype A1,
    (b') un extrait contenant des antigènes capsulaires et/ou de membrane de Pasteurella haemolytica sérotype A1,
    (c') des corps bactériens entiers de Pasteurella haemolytica sérotype A1, et
    (d') des fractions de tels corps bactériens, ledit surnageant, ledit extrait, lesdits corps bactériens et lesdites fractions étant biologiquement inactivés.

**3.** Vaccin selon la revendication 1 ou 2, caractérisé en ce que ladite première substance antigène est constituée par le surnageant d'une culture de Pasteurella haemolytica sérotype A6, ce surnageant étant concentré et biologiquement inactivé.

**4.** Vaccin selon la revendication 2 ou 3, caractérisé en ce que ladite seconde substance antigène est constituée par le surnageant d'une culture de Pasteurella haemolytica sérotype A1, ce surnageant étant concentré et biologiquement inactivé.

**5.** Vaccin selon l'une quelconque des revendications précédentes, caractérisé en ce qu'il comprend en outre un ajout de leucotoxine de Pasteurella haemolytica, autre que la leucotoxine éventuellement déjà présente dans ladite première substance antigène et/ou ladite seconde substance antigène.

**6.** Vaccin selon l'une quelconque des revendications précédentes, caractérisé en ce que l'extrait contenant des antigènes capsulaires de Pasteurella haemolytica sérotype A6 et l'extrait contenant des antigènes capsulaires de Pasteurella haemolytica sérotype A1 sont respectivement ceux obtenus par extraction de cellules de Pasteurella haemolytica sérotype A6 ou de Pasteurella haemolytica sérotype A1 par une solution aqueuse d'un sel minéral ou organique.

**7.** Vaccin selon l'une quelconque des revendications précédentes, caractérisé en ce que ledit (lesdits) surnageant (s), extrait(s) et corps bactériens entiers et lesdites fractions de corps bactériens ont été inactivés par le formaldéhyde ou le phénol.

**8.** Vaccin selon l'une quelconque des revendications précédentes, caractérisé en ce qu'il comprend en outre au moins un adjuvant d'immunisation.

**9.** Vaccin selon la revendication 8, caractérisé en ce que ledit adjuvant d'immunisation est choisi dans le groupe constitué par l'hydroxyde d'aluminium sous forme de gel et une saponine.

**10.** Concentré du surnageant d'une culture bactérienne de Pasteurella haemolytica sérotype A6, ce concentré ou ce surnageant étant inactivé ou non.

**11.** Procédé de préparation du vaccin selon l'une quelconque des revendications 1 à 9 dans lequel la première et la seconde substances antigènes sont des surnageants concentrés de culture bactérienne, caractérisé en ce qu'il comprend les opérations de :

   (a) culture d'une souche-mère de Pasteurella haemolytica sérotype A6,
   (b) culture séparée d'une souche-mère de Pasteurella haemolytica sérotype A1,
   (c) séparation par filtration d'une partie au moins des cellules bactériennes du surnageant obtenu par l'opération (a) ci-dessus,
   (d) séparation par filtration d'une partie au moins des cellules bactériennes du surnageant obtenu par l'opération (b) ci-dessus,
   (e) concentration du surnageant obtenu par l'opération (c) et du surnageant obtenu par l'opération (d) ci-dessus,
   (f) mélange des concentrés obtenus par l'opération (e) ci-dessus dans une proportion appropriée,
   (g) inactivation biologique des deux concentrés obtenus par l'opération (e) ci-dessus, avant ou après leur mélange par l'opération (f) ci-dessus,
   (h) adjonction éventuelle d'un ou plusieurs adjuvants d'immunisation au mélange de concentrés inactivés obtenu précédemment,
   (i) adjonction éventuelle de leucotoxine de Pasteurella haemolytica, et
   (j) réglage éventuel du pH du mélange obtenu par l'opération (i) ci-dessus à la valeur souhaitée.

**12.** Procédé de préparation du vaccin selon l'une quelconque des revendications 1 à 9 dans lequel la première et la seconde substances antigènes sont des extraits contenant des antigènes capsulaires, caractérisé en ce qu'il comprend les opérations de :

   (a) culture d'une souche-mère de Pasteurella haemolytica sérotype A6 et culture séparée d'une souche-mère de Pasteurella haemolytica sérotype A1,
   (b) collecte des cellules bactériennes obtenues par l'opération (a) ci-dessus,

(c) extraction par une solution aqueuse d'un sel minéral ou organique respectivement des cellules collectées à l'opération (b),

(d) élimination des matières cellulaires pour récupérer un extrait aqueux contenant des antigènes capsulaires de Pasteurella haemolytica sérotype A6 et un extrait aqueux contenant des antigènes capsulaires de Pasteurella haemolytica sérotype A1,

(e) purification de chacun des extraits,

(f) mélange des extraits purifiés,

(g) inactivation biologique desdits extraits avant ou après leur mélange par l'opération (f) ci-dessus,

(h) adjonction éventuelle d'un ou plusieurs adjuvants d'immunisation au mélange des extraits inactivés obtenu précédemment,

(i) adjonction éventuelle de leucotoxine de Pasteurella haemolytica, et

(j) réglage éventuel du pH du mélange obtenu par l'opération (i) ci-dessus à la valeur souhaitée.

## RAPPORT DE RECHERCHE EUROPEENNE

Office européen
des brevets

Numéro de la demande

EP 98 40 0606

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | MORTON ET AL.: "Vaccination of cattle with outer membrane protein-enriched fractions of Pasteurella haemolytica and resistance against experimental challenge exposure" AM. J. VET. RES., vol. 56, no. 7, 1995, pages 875-879, XP002050680 * le document en entier * | 1-12 | A61K39/102 C07K16/12 |
| X | WELLS ET AL.: "Development of a combined clostridial and Pasteurella haemolytica vaccine for sheep" THE VETERINARY RECORD, vol. 114, 1984, pages 266-269, XP002050681 Page 266, la colonne à droite, "Vaccines" * le document en entier * | 1-12 | |
| X | EVANS AND WELLS: "A mouse model of Pasteurella haemolytica infection and its use in assessment of the efficacy of P haemolytica vaccines" RESEARCH IN VETERINARY SCIENCE, vol. 27, 1979, pages 213-217, XP002050682 * le document en entier * | 1-12 | DOMAINES TECHNIQUES RECHERCHES (Int.Cl.6) A61K |
| X | DONACHIE ET AL.: "Comparison of cell surface antigen extracts from two serotypes of Pasteurella haemolytica" JOURNAL OF GENERAL MICROBIOLOGY, vol. 130, 1984, pages 1209-1216, XP002050683 * le document en entier * | 1-12 | |

-/--

Le présent rapport a été établi pour toutes les revendications

| Lieu de la recherche | Date d'achèvement de la recherche | Examinateur |
|---|---|---|
| LA HAYE | 9 juillet 1998 | Hagenmaier, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**Office européen des brevets**

# RAPPORT DE RECHERCHE EUROPEENNE

Numéro de la demande

EP 98 40 0606

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | GB 2 023 420 A (HOECHST UK LTD) 3 janvier 1980<br>* le document en entier * | 1-12 | |
| A | SHEWEN AND WILKIE: "VACCINATION OF CALVES WITH LEUKOTOXIC CULTURE SUPERNATANT FROM PASTEURELLA HAEMOLYTICA"<br>CANADIAN JOURNAL OF VETERINARY RESEARCH, vol. 52, no. 1, janvier 1988, pages 30-36, XP002070565<br>* le document en entier * | 1-12 | |
| A | CONLON ET AL.: "EFFICACY OF RECOMBINANT LEUKOTOXIN IN PROTECTION AGAINST PNEUMONIC CHALLENGE WITH LIVE PASTEURELLA HAEMOLYTICA A1"<br>INFECTION AND IMMUNITY, vol. 59, no. 2, 1991, pages 587-591, XP002070566<br>* le document en entier * | 1-12 | |
| A | WO 80 00412 A (GILMOUR N ;THOMPSON D (GB); MARTIN W (GB); WELLS P (GB); SHARP J () 20 mars 1980<br>* le document en entier * | 1-12 | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 9 juillet 1998 | Hagenmaier, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)